# EUROPEAN PATENT SPECIFICATION

(11) **EP 1 830 715 B1**
(45) Date of publication and mention of the grant of the patent: **07.12.2016**
(21) Application number: 05820771.3
(22) Date of filing: 25.10.2005
(51) Int. Cl.: A61B 17/04, A61B 17/11

(54) **SUTURE GUIDE**
WUNDNAHTFÜHRUNG
GUIDE DE SUTURE

(30) Priority: 25.10.2004 US 621720 P
(43) Date of publication of application: 12.09.2007
(73) Proprietor: Deland, Jonathan T., New York NY 10021 (US)
(72) Inventor: Deland, Jonathan T., New York NY 10021 (US)
(74) Representative: O'Connor, Donal Henry
(86) International application number: PCT/US2005/038490
(87) International publication number: WO 2006/047563

(56) References cited:
- US-B1- 6 322 571

## Description

### BACKGROUND

Tendon rupture is a debilitating event that limits motion and can cause pain. Rupture can result from overexertion, trauma, and age-related degeneration, among other causes. Surgical repair of the ruptured tendon is typically required; tendinous tissue has poor wound-healing properties, and the torn ends of the tendon separate from one other due to contraction of the unrestrained muscle attached to one tendon end.

Surgical repair of a ruptured tendon is typically performed by putting one or more sutures through each torn end and then sewing the complementing sutures to one another, thereby winching the torn ends together and restoring the connected muscle to its normal resting length. Two risks of tendon repair include inadequate strength of the repair and potential soft tissue problems from surgical exposure. Accordingly, it would be best if a tendon could be repaired through a small incision but with a strong repair. With a small incision, the needles used to advance the sutures through the tendon may be advanced manually, without any guides, but this practice risks placing the sutures unevenly, so that the tendon's natural geometry and strength are not restored, and the repair is weak. One approach, described in U.S. Pat. No. 6,200,327 to Assal, provides a two-piece guide member with aligned channels in each piece. The aligned channels allow a user to advance a loaded needle horizontally through the tendon in a precise and repeatable fashion. However, the structure of the Assal device necessarily limits each suture to just one pass through the tendon; this results in a potentially weak stitch that provides a minimum of surface area for the suture to engage the tendon.

US 6,322,571 discloses a suture repair system for releasably holding the lacerated end of a tendon, ligament or the like body tissue and precisely guiding placement of sutures therein. The system includes a suture guide path for placing sutures in the lacerated end.

### SUMMARY

The present disclosure describes the structure and use of various suture guides that facilitate the precise and reproducible placement of multiple passes of a suture through a tissue.

The scope of the invention is as defined by the appended claims.

In the main embodiment of the invention as defined in claim 1, a suture guide comprises:
a tissue guard; and
a flanking post disposed to one side of the tissue guard, the flanking post defining a plurality of channels passing through its width, the channels so oriented that:
   a) the channels define suture needle guide paths in one or more planes that do not intersect the tissue guard; and
   b) at least one channel is not perpendicular to a long axis of the flanking post, so that it defines an oblique suture needle guide path;
characterized in that the post and the guard are attached to one another through a connecter so adjustable as to control the spacing between the post and the guard.

In another embodiment the suture guide further comprises a shaft to which the guard and post are so coupled as to make the post and the guard rotatable with respect to one another. In another embodiment the guard has a concave shape, the concavity faces the suture needle guide paths, and edges of the guard concavity extend backward to, but not into, the forward-most suture needle guide plane.

In another embodiment the flanking post defines at least one channel oriented in a first direction not perpendicular to the long axis of the flanking post, and at least one additional channel oriented in a second direction, different from the first direction, not perpendicular to the long axis of the flanking post.

In another embodiment at least one channel of the flanking post is perpendicular to the long axis of the flanking post so that it defines a horizontal suture needle guide path.

In another embodiment the suture guide further comprises a second flanking post disposed to the side of the guard opposite from that of the first flanking post, each flanking post defining a plurality of channels passing through its width, the channels so oriented that:
a) the channels define suture needle guide paths in one or more planes that do not intersect the tissue guard; and
b) at least one channel is not perpendicular to the long axis of the flanking post so that it defines an oblique suture needle guide path.

In another embodiment the channels of the two flanking posts are so aligned that one non-perpendicular channel from each of the first and second flanking posts are coaxial and together define an oblique suture needle guide path through the first post and the second post.

In another embodiment at least one channel of each flanking post is perpendicular to the long axis of the flanking post so that it defines a horizontal suture needle guide path.

### BRIEF DESCRIPTION OF THE DRAWINGS

**FIGS. 1-5, 11-13,** and **16** show perspective views of various exemplary suture guides.
**FIGS. 6-8** show top plan views of exemplary suture guides.
**FIGS. 9-10** and **14-15** show side elevation views of exemplary suture guides.

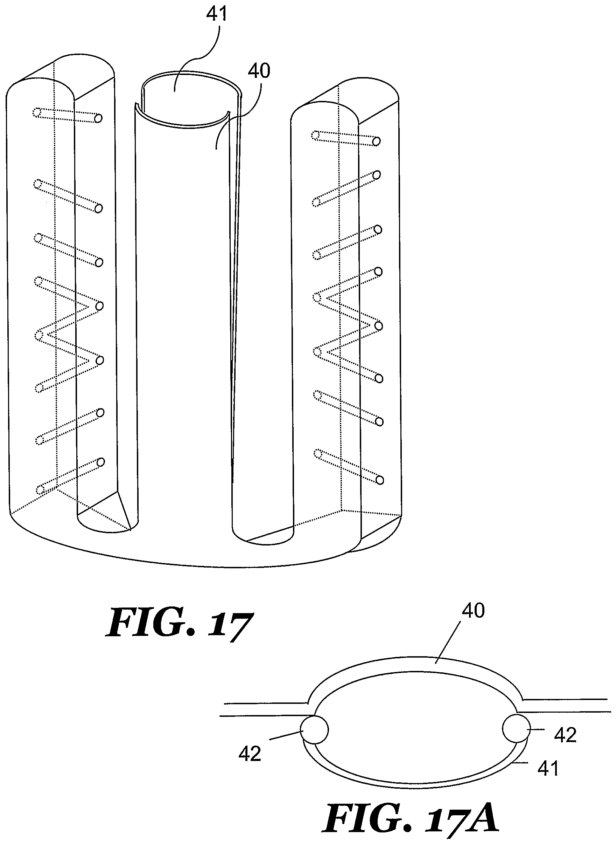

**FIGS. 17-17A** show perspective and elevation views, respectively, of another exemplary suture guide.
**FIGS. 18-32** depict exemplary uses of suture guide.

### DETAILED DESCRIPTION

The suture guides disclosed herein provide suture needle guide paths that assist a surgeon or other user in positioning stitches in a precise and reproducible way. In particular, the disclosed suture guides include needle guide paths that allow the user to create multiple passes of a stitch through a tissue, which are stronger than a single pass.

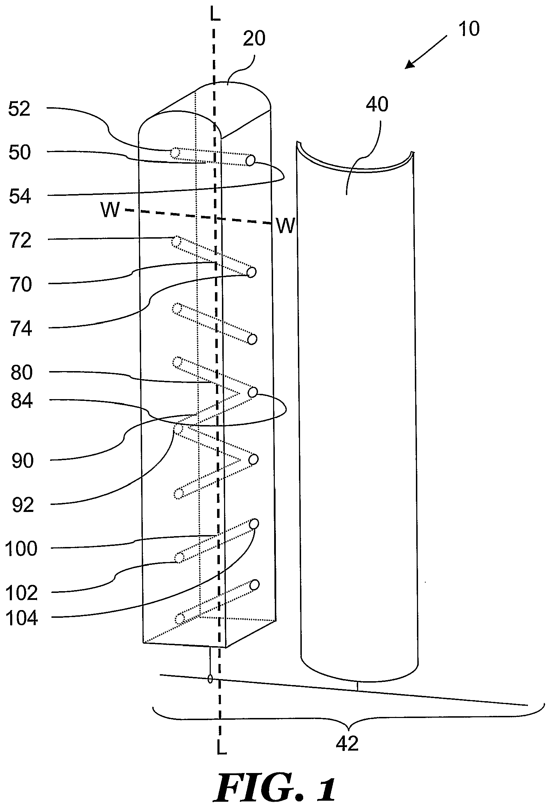

**FIG.1** shows one embodiment of a suture guide **10.** In this embodiment, the suture guide includes a tissue guard **40** and a flanking post **20.** In the depicted embodiment, the tissue guard has a concave shape, and the concavity faces toward the back of the device. This shape can facilitate a snug contact between the guard and an underlying convex tissue. The guard may various degrees of concavity, from, for example, about 120 degrees of arc to about 180 degrees of arc. The guard may also have other shapes, including a flat plate, rounded corners, squared-off corners, v-shaped, and others. The free end of the guard (the tip) may be curved, tapered, rounded, and/or thinned, akin to the blades of a vaginal speculum, to facilitate sliding along tissue.

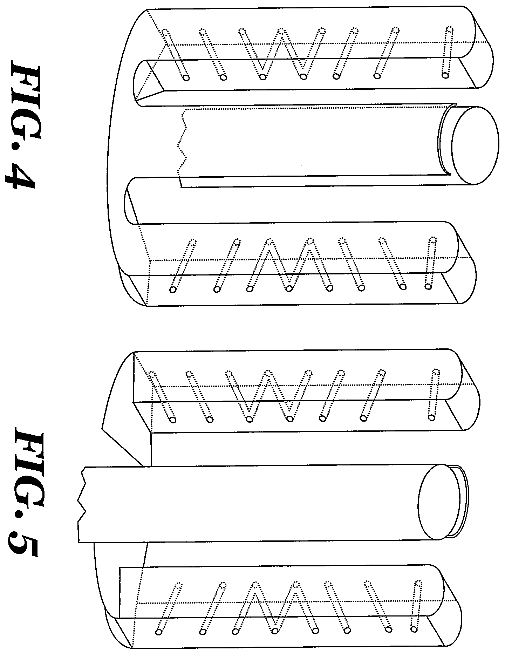

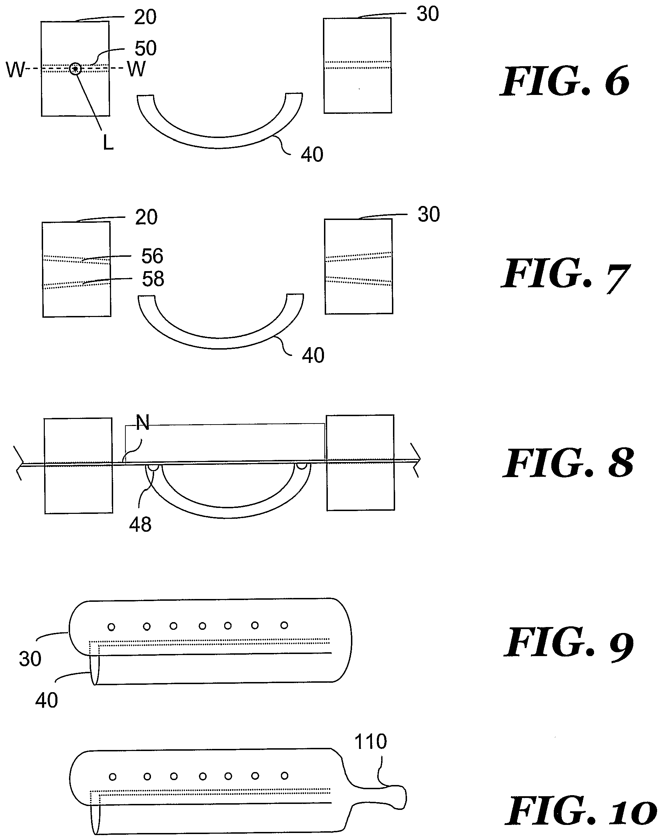

The flanking post is disposed to one side of the tissue guard. The post defines a plurality of channels **50, 70, 80,** etc. that extend through the post's width, from external orifices **52, 72, 92,** etc. to internal orifices **54, 74, 84,** etc. The channels are positioned so that a needle passing through one of the channels will move in a plane that does not intersect the tissue guard. This detail is shown more clearly in **FIGS. 6-10****.** When the device is positioned on a tissue, the needles will then pass through the tissue behind (**FIG. 4**) or in front (**FIG. 5**) of the guard without being obstructed by the guard. "Behind" and "front" in this sense refer to the guard as viewed in the figures. When a suture guide is positioned for an Achilles tendon repair, for example, the needles will pass anterior or posterior to the tissue guard, where "anterior" and "posterior" are defined with respect to the patient's anatomy.

One or more of the channels may be parallel to the width of the flanking post, *i.e.,* parallel to an axis **W** through the width of the post, to ensure that a needle passing through them will not intersect the tissue guard. However, one or more channels can be oriented non-parallel so that sutures passing through the tissue are in different planes and do not interfere with one another. The non-parallel channel should be so angled that its axis does not intersect the tissue guard. **FIG. 7** shows one embodiment having non-parallel channels **56, 58.**

One or more of the channels, exemplified here as channel **50,** may be perpendicular to the long axis **L** of the flanking post in addition to being parallel to the post's width. This channel can define a horizontal suture needle guide path. (The path does not appear horizontal in the drawing due to the perspective.) At least one other channel, such as channel **70,** is not perpendicular to the post's long axis, although it is still parallel to the width. This channel defines an oblique suture needle guide path. Some of the oblique channels may run in a first non-perpendicular direction, such as channels **70** and **80,** while other oblique channel may run in a second non-perpendicular direction different fro the first direction, such as channels **90** and **100.** Adjacent oblique channels running in different directions can share a common orifice, such as orifice **84** shared by channels **80** and **90.** It should be noted that the depicted post embodiment defines nine channels, but alternative embodiments may have more or fewer channels in accordance with particular applications.

In this embodiment, the post and the tissue guard are connected to one another by a rod **42.** The guard can be affixed to the connector, while the post can be clampable and repositionable. For example, the post could be clamped in the illustrated position during part of a suturing procedure, then moved to the other side of the rod during another part of the procedure. A sliding arrangement can also allow the post to be positioned an appropriate distance from the tissue guard for a given tissue size or to accommodate the local anatomy.

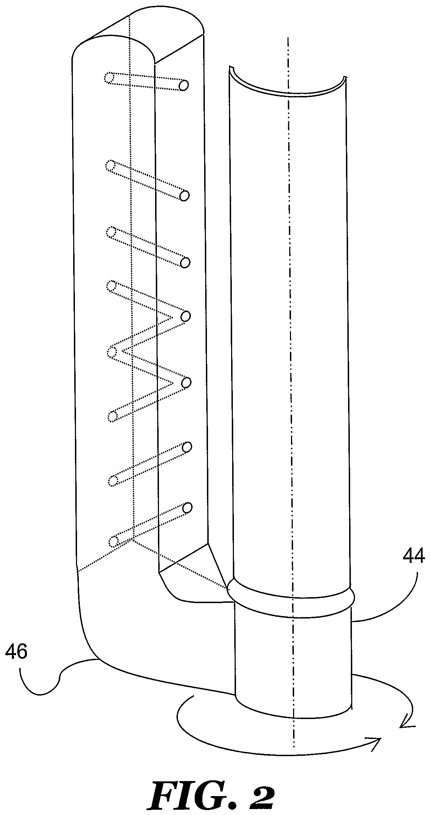

**FIG. 2** shows another exemplary suture guide similar to the embodiment of
**FIG. 1****,** except that instead of a connecting rod, the guide has a shaft **44** to which the post is rotatably mounted by arm **46.** The post can rotate about the shaft and thereby swing from one side of the guard to the other side. The post and/or shaft can include a mechanism to help ensure that the post is rotated into an appropriate position, *i.e.,* one in which the tissue guard will not obstruct needles passing through the channels. Examples of appropriate mechanisms include a paired notch and detent, a stop, a clasp, a clamp, and alignment markings on the post and/or shaft to guide the user. The tissue guard may also be rotatable about the shaft, so that it can be swiveled between an anterior position and a posterior position.

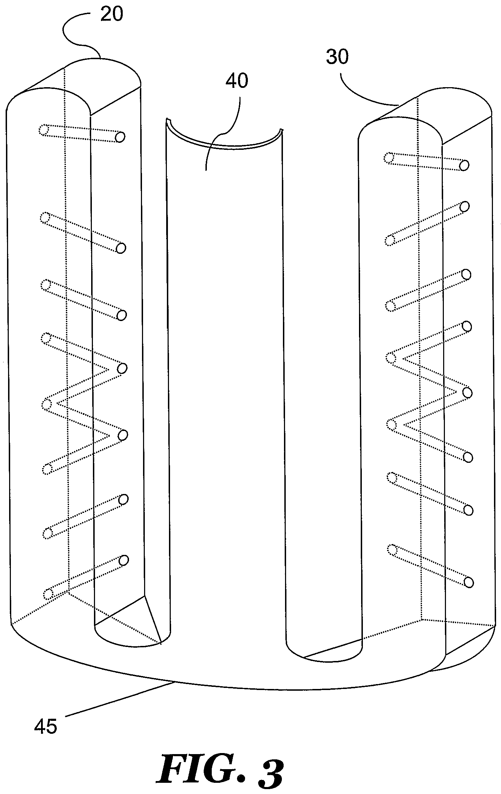

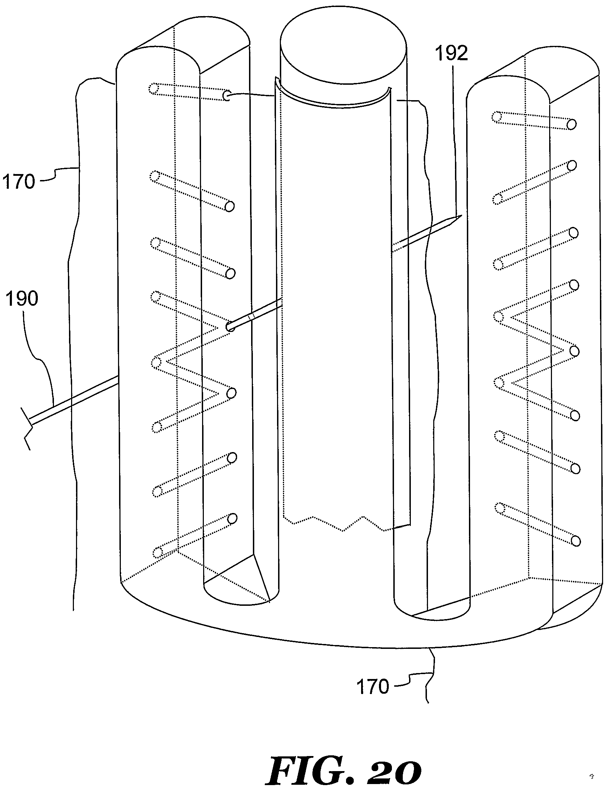

**FIG. 3** shows yet another exemplary suture guide which includes a second flanking post **30** disposed to the side of the guard opposite from that of the first flanking post. The second flanking post, as illustrated, is a mirror image of the first post and includes a plurality of channels that complement the channels of the first post. It is preferred that the channels are so aligned that a needle passing through a channel in the first post will glide smoothly into a similarly-directed channel in the second post. An example of this is shown in **FIGS. 20-21****.** In the example depicted in **FIG. 3****,** the posts are connected to the guard by a base **45.** The base, guard, and post or posts can be of unitary construction. Alternatively, the parts could be connected by an adjustable connecter as described earlier, such as a rod/clamp, or a bolt/screw arrangement, so that the posts may be positioned on either side of the guard in the optimal positions for a given use.

The disclosed suture guides can also be so positioned that the tissue guard supports tissue rather than covers it. This can be done by turning the device over and slipping the guard behind the tissue to be sutured. The guard would help hold the tissue to be sutured away from surrounding tissue and would also protect tissue deep to the tissue to be sutured.

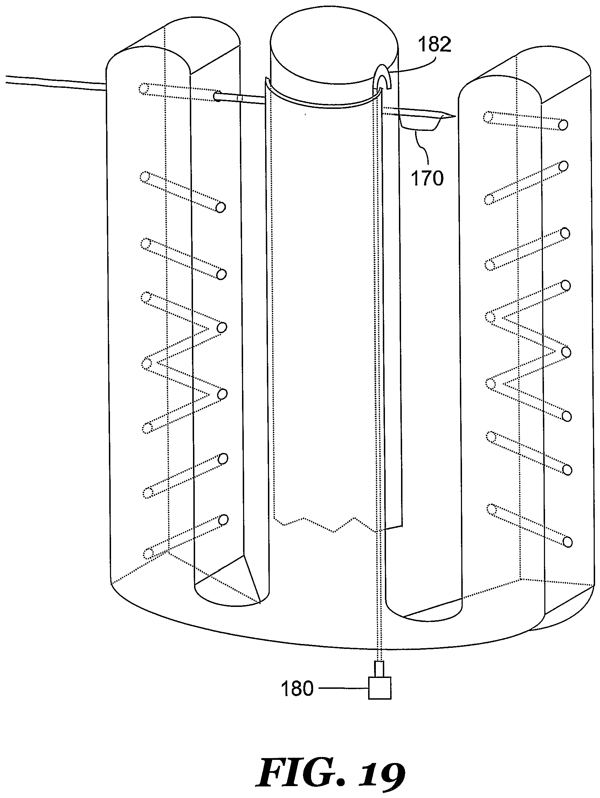

**FIGS. 6-10** show additional views of various exemplary suture guides to depict certain features more clearly. **FIG. 6** is a transverse cross section of the **FIG. 3** example taken at the level of channel **50** and projected upward. Width axis **W** and long axis L are depicted. Tissue guard **40** is positioned so that the suture needle guide paths are situated in one or more planes behind the guard, so that needles advanced through those paths will enter tissue held behind the guard during use. The guard does not extend so far back as to block the needle path planes, as this would interfere with suturing. However, as shown in **FIG. 8****,** the guard may extend backward just up to the foremost guide plane, so that the edge of the guard may further assist needle **N** guidance. **FIG. 8** also shows an alternative example of guard 40, in which the edges of the guard define grooves **48** that may be used to slide suture catches up and down the guard. This feature will be described in more detail with reference to **FIG. 19****.** **FIGS. 9-10** show side view of suture guides.
**FIG. 10****,** in particular, shows a suture guide with handle **110** to simplify the grip and positioning of the device.

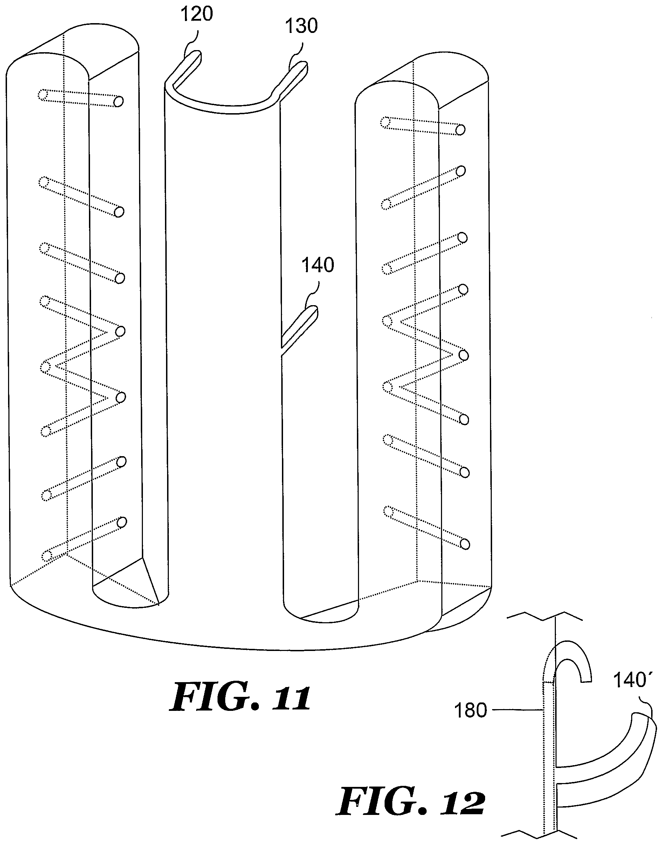

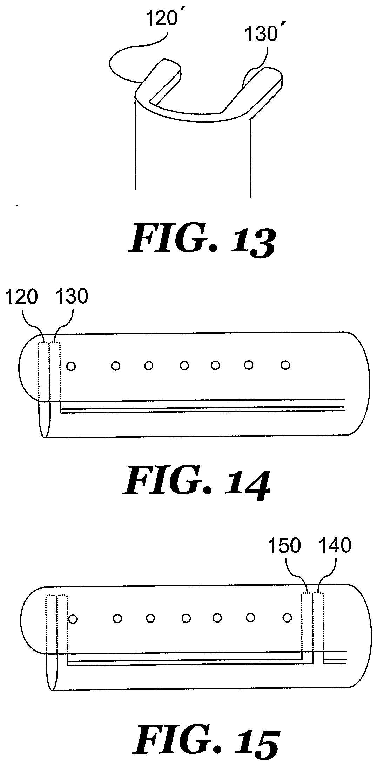

**FIGS. 11-15** show exemplary alternatives of suture guides that include additional protuberances on the guard. The guard depicted in **FIG.11** includes catch receptacles **120** and **130.** These protuberances prevent suture catches that are slid along the guard from advancing beyond the guard and potentially damaging tissue beyond the guard. In some examples, a guard may include a protuberance **140** disposed along the guard. A complementary guard on the other side of the guard is not visible in this perspective view, but is better seen as protuberance **150** in **FIG. 15****.** The protuberances **140** and **150** help hold away body structures that are deeper than the tissue being sutured, to prevent the suture catch from inadvertently contacting the deep structure, and also to keep the deep structure away from the suture needle guide paths.

It is preferred that the mid-guard protuberances be so positioned that they do not cross the suture needle guide paths, so that they do not interfere with use of the device. It is also preferred that the mid-guard protuberances do not interfere with the movement of suture catches along the tissue guard. In some cases, as shown in **FIG. 12****,** the protuberance **140'** can be shaped so that its body is clear of the guard and suture catch **180** (discussed in more detail with reference to **FIG. 19**) can pass unimpeded. **FIG. 13** shows detail of an alternate example, in which the catch receptacles **120'** and **130'** are larger than the previous example. The receptacles should be blunt, not sharp, so that they do not poke surrounding tissue. **FIGS. 14** and **15** show side views of examples that include catch receptacles and/or mid-guard protuberances. (Note that, as shown in **FIGS. 14-15****,** the sides of the tissue guard need not extend into the space between the posts as they do in **FIGS. 6-7****.**)

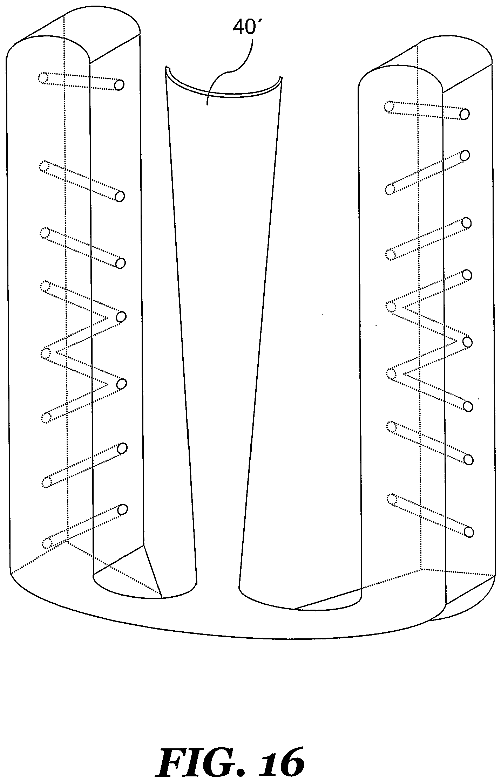

**FIG. 16** shows another exemplary suture guide in which the guard **40'** is tapered along its length. The guard may gradually narrow as it approaches the guide base. This tapered shape may be preferred when the device is used to suture a tendon or other tissue which itself has a tapered shape.

The suture guide can be formed from a variety of materials, including metals and plastics. A disposable, single-use device can be made from plastic and have a unitary design, so that it could be made by injection molding. A sterilizable, reusable device can be made from metal, such as stainless steel or other metals typically used in surgical applications.

**FIGS, 17-17A** depict another exemplary suture guide, which includes a second tissue guard **41** pivotably coupled to the other tissue guard by hinges **42.** The hinges may be lockable by, for example, a ratchet or a screw (not shown). The tips of the blades may be adapted for sliding along tissue, as disclosed above. The two guards permit simultaneous protection of tissues above and below the tissue being sutured using the suture guide. To position the double-guard device, the guards are swung to an open position, and the device is slid over the tissue like a sleeve. The guards can then be swung against the tissue and tightened, if desired.

### EXAMPLES

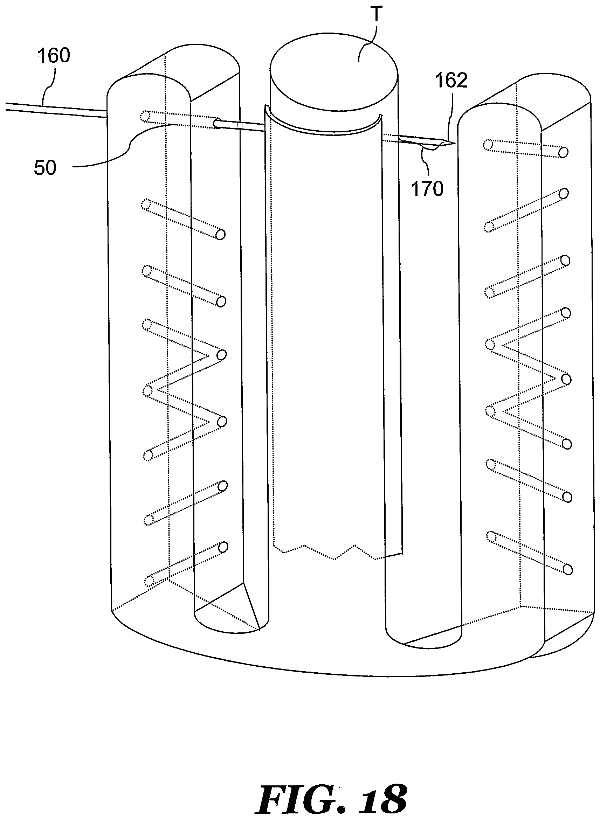

**FIGS. 18-32** describe, step-by-step, exemplary uses of suture guides. These examples depict the use of a suture guide having two posts, but the depicted uses can also be accomplished using a one-post suture guide, in which the device is flipped over, or the post repositioned, when maneuvers involving the second post are called for. These figures are schematic representations and are not necessarily depicted to scale; rather, they illustrate the principles of use. The end result of the exemplary uses is to have a suture threaded through the tissue in a crisscross pattern.

**FIG. 18** shows the starting condition for an exemplary suturing procedure. The suture guide is placed over a tissue **T** to be sutured so that the guard covers the tissue and the flanking posts lie on either side of the tissue. (In another example, a device could be turned over, so that the tissue guard supports the tissue.) A first needle **160** is advanced through the horizontal channel **50,** emerges into the space between the posts, and then enters the tissue. The horizontal channel positions the needle for a horizontal trajectory through the tissue. The needle may have a sharp tip **162** to facilitate clean entry of the needle into the tissue. As the needle passes through the tissue, it forms a horizontal needle track. The needle **160** is loaded with a first suture **170;** the suture is drawn through the horizontal track. Once the needle emerges on the far side of the tissue, the first suture is accessible on the second side of the tissue. (In alternate example, the horizontal channel might not be used or might be omitted from the device.)

Next, shown in **FIG. 19****,** a suture catch **180** maybe advanced up through the guard. The catch may be guided up the suture through a groove, such as groove **48 (****FIG. 8****).** The catch may have a hook **182** or similar structure to catch a suture. The hook may be curved to various extents. The hooks shown are curved through about 180 degrees, but hooks may be curved less, such as about 120 degrees, or may be curved more, such as through about 270 degrees, about 300 degrees, about 330 degrees, about 350 degrees, or about 360 degrees, so that the hook more resembles an eye or a loop. The hook is maneuvered to catch the suture (or may have been already placed in position before the passage of the first suture in **FIG. 18**), and then the catch is pulled down through the guard, taking the first suture with it. The catch may then be advanced again up through the guard to be in position for the next suture.

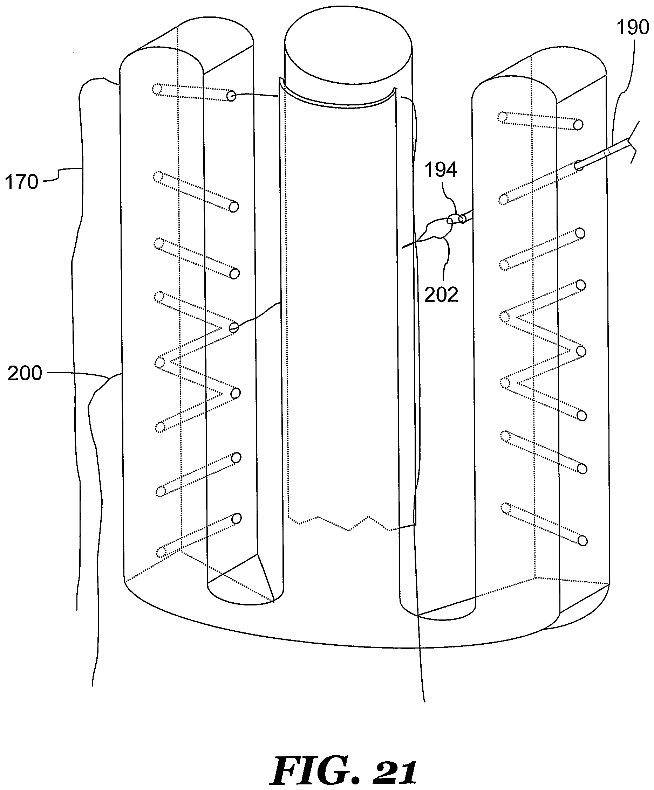

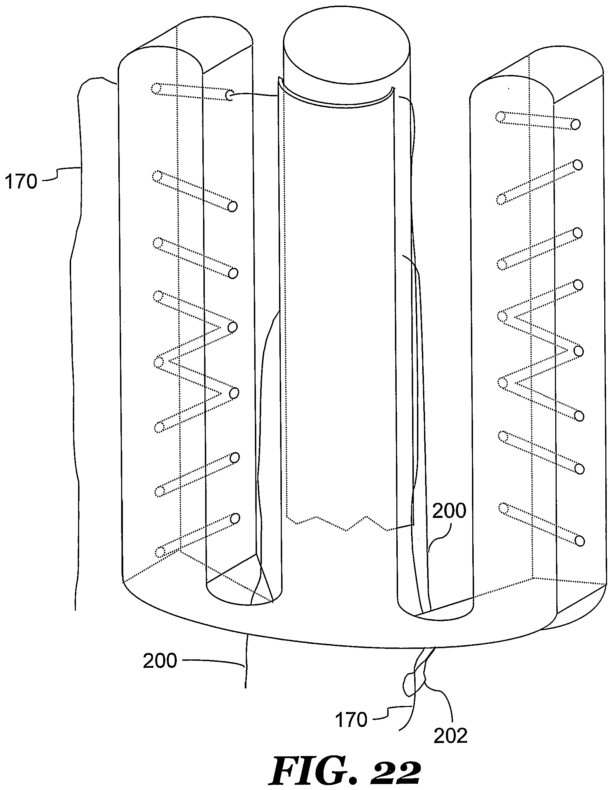

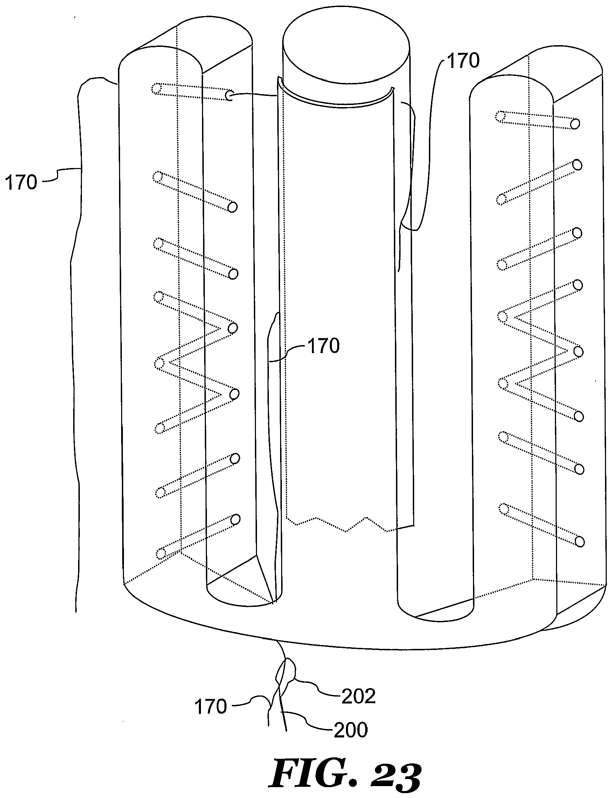

In **FIG. 20****,** a second needle **190** is advanced through one of the oblique guide paths of the first post, into the tissue, out the far side of the tissue, and, optionally, into the corresponding channel in the second post. This motion forms an oblique track through the tissue. As shown in **FIG. 21****, a** second suture **200** is attached to the back of the second needle. The second suture has a catch on its front end, such as a loop **202,** and the second suture may be attached to the second needle by another loop **194** attached to the back of the needle. Once the front end of the second suture **200** emerges on the far side of the tissue, it is detached from the second needle, hooked by the suture catch **180** (**FIG. 19**), and brought down through the guard. The back end of the second suture protrudes from the first side of the tissue so that it can be grasped later. Next, shown in **FIG. 22****,** the first suture **170** is threaded through the catch **202** on the second suture. In some examples, the catch could be tightened like a noose to grasp the first suture. Alternatively, the first and second sutures could be knotted or otherwise bonded. The back end of the second suture is then pulled so that the second suture is retracted back through the oblique track and pulls the first suture along with it. **FIG. 23** shows the end of this step, with the first suture now making one horizontal pass through the tissue and one oblique pass. The distance between the exit point of the horizontal pass and the entry point of the oblique pass may vary; it is exaggerated in the drawing to show detail. In practice, such as during a repair of the Achilles tendon, the separation between the exit and entry points may be in the range of about 6 millimeters to about 26 millimeters. The separation can vary depending on factors such as the size or consistency of the tissue to be sutured (for example, other tendons or other tissue types), the location or extent of the rupture, or the particular anatomy of the subject. The separation distance can be controlled, for example, by providing several suture needle guide paths from among which the user can select, or by instructing the user to reposition the suture guide.

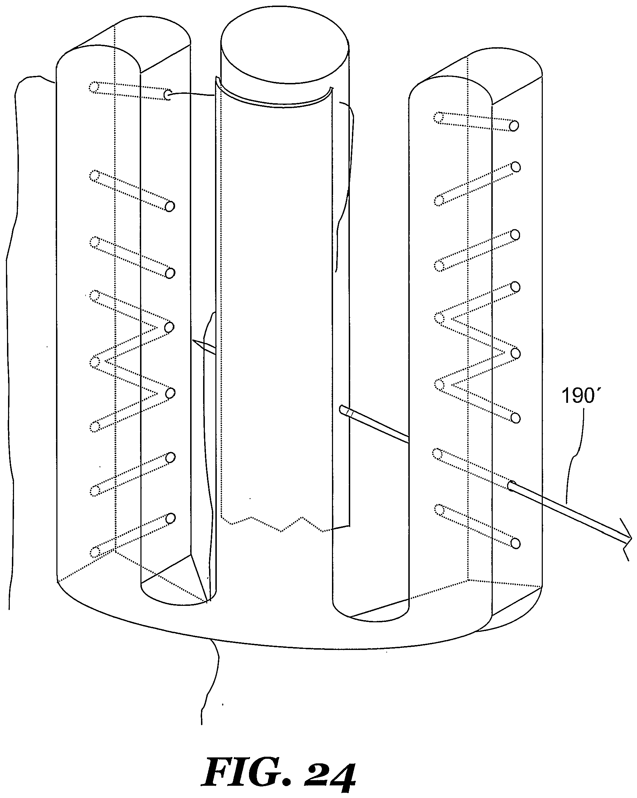

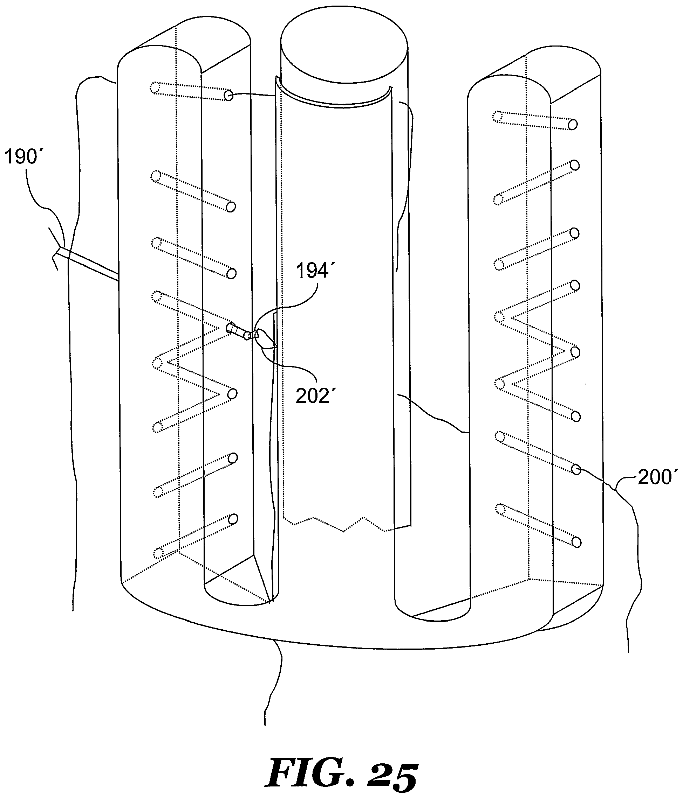

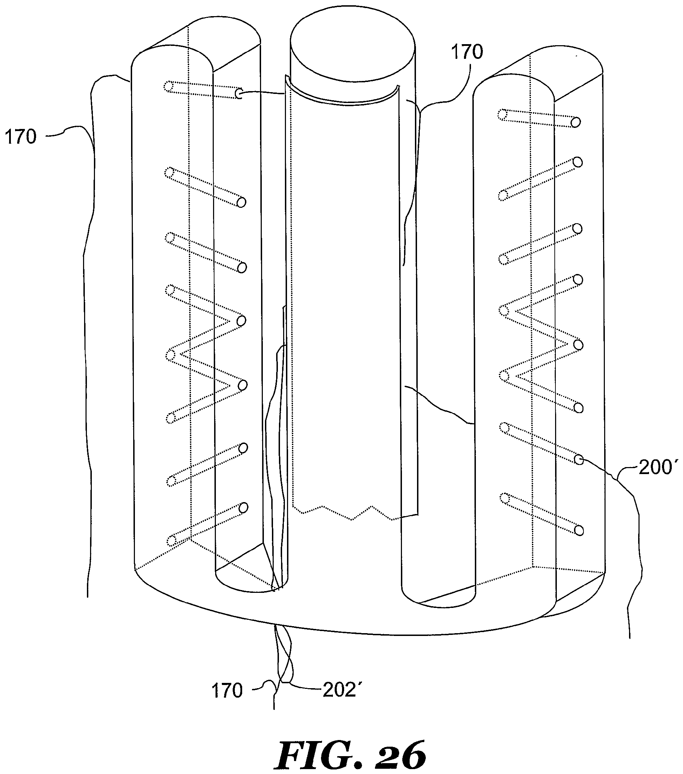

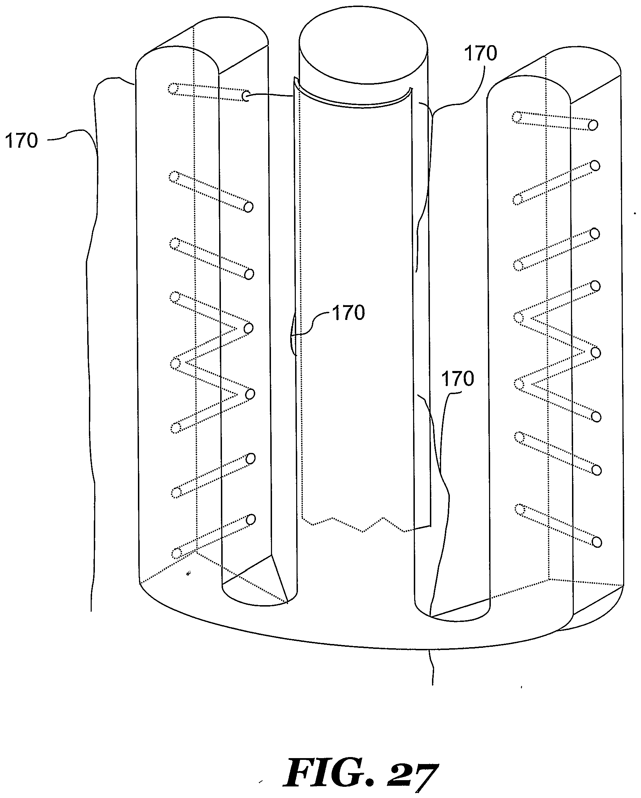

**FIG. 24** shows the next step. The second needle is reloaded with the second suture or another suture. Alternatively, a third needle, with its own suture is provided. This needle **190'** is advanced in an oblique channel from the other side of the tissue. If the suture guide has two posts, this channel would be in the second post. If the guide has one post, that post would be moved to the other side. This needle is advanced all the way through the tissue (**FIG. 25**), thereby forming a second oblique needle track, and brings its suture **200'** out to the first side of the tissue. The suture **200'** is detached from the needle by cutting link **194'.** A suture catch is advanced along the guard to hook the suture and bring its catch **202'** down, and, as shown in **FIG. 26****,** the first suture **170** is threaded through it, as before. The suture **200'** is then backed out through the second oblique needle track, pulling the first suture **170** with it. **FIG. 27** shows the state of the procedure at this point: the first suture **170** has now made three passes through the tissue.

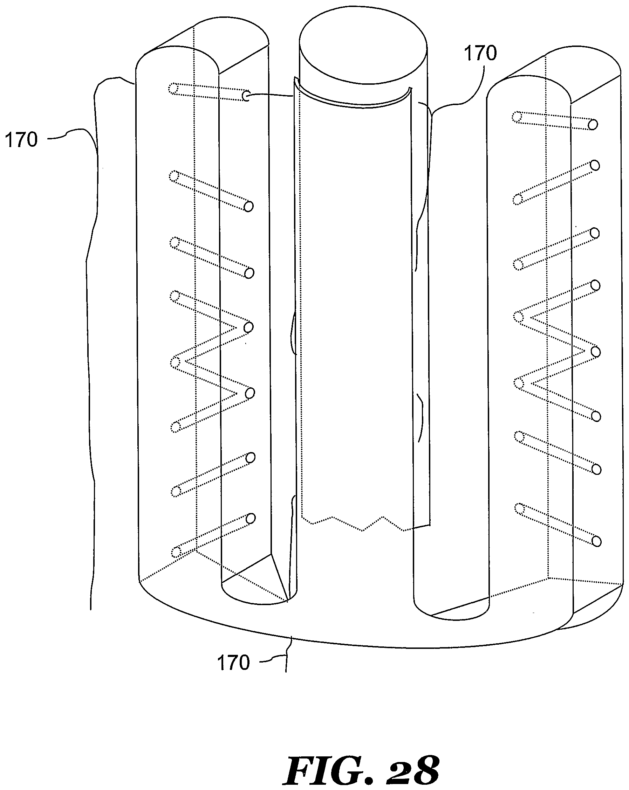

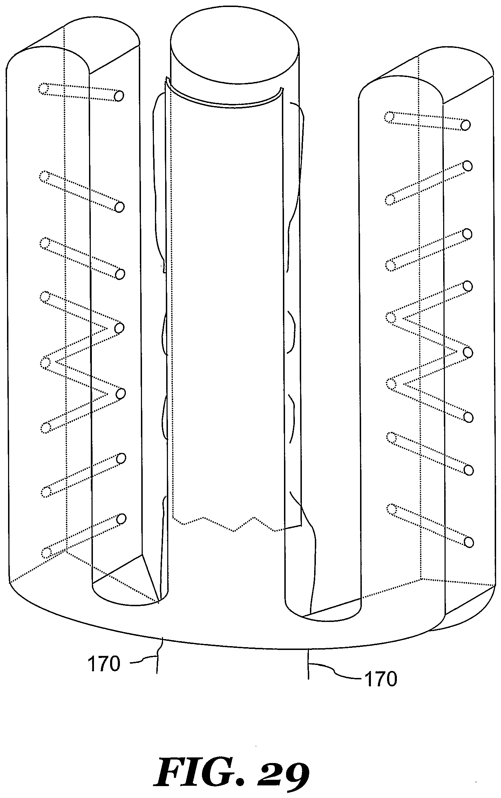

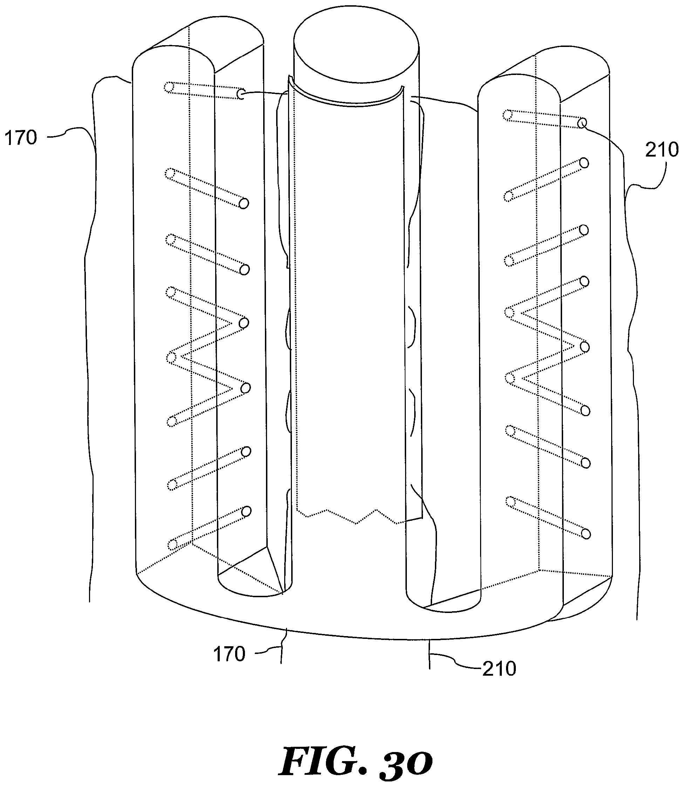

The process may be repeated one or more times to give the suture additional oblique passes through the tissue. **FIG. 28** shows the condition after one additional oblique pass is formed. The dangling end of the first suture **170** may be pulled into the space between the first post and the guard using a catch **180,** and the entire procedure repeated, so that both ends of the first suture are stitched through the tissue in a crisscross pattern. The end result of this procedure is shown in **FIG. 29****.** Alternatively, or in addition, a second suture **210,** shown in **FIG. 30****,** can be introduced through a channel in the second post (or relocated single post) and stitched through the tissue in mirror image to the first suture **170.** As many additional sutures as desired can be placed in the tissue in this manner.

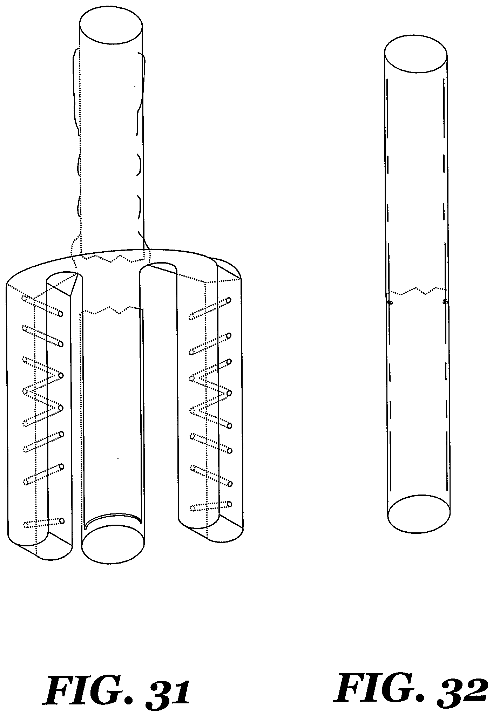

The entire process may then performed on the other end of the ruptured tissue, as shown in **FIG. 31**. In some examples, sutures could be passed through the other ruptured tissue end using conventional techniques. The free ends of sutures placed in each tissue end are pulled together and tied, as shown in **FIG. 32****,** thereby completing the tissue repair.

Exemplary uses of the disclosed suture guides include Achilles tendon repair, anterior tibial tendon repair, repair of extensor tendons, flexor tendons to the fingers, tendons of the arm as well as other tendons and other tissue types.

## Claims

1. A suture guide (10), comprising:
a tissue guard (40) configured to overlie or support tissue ; and
a flanking post (20) disposed to one side of the tissue guard (40), the flanking post (20) defining a plurality of channels (50, 70, 80, 90, 100) passing through its width, the channels (50, 70, 80, 90, 100) so oriented that:
a) the channels (50, 70, 80, 90, 100) define suture needle guide paths in one or more planes that do not intersect the tissue guard (40); and
b) at least one channel (70, 80, 90, 100) is not perpendicular to a long axis (L) of the flanking post (20), so that it defines an oblique suture needle guide path;
**characterised in that** the post (20) and the guard (40) are attached to one another through a connecter (42) so adjustable as to control the spacing between the post (20) and the guard (40).

2. The suture guide (10) of claim 1, further comprising a shaft (44) to which the guard (40) and post (20) are so coupled as to make the post (20) and the guard (40) rotatable with respect to one another.

3. The suture guide (10) of any preceding claim, wherein the guard (40) has a concave shape, the concavity faces the suture needle guide paths, and edges of the guard concavity extend backward to, but not into, the forward-most suture needle guide plane.

4. The suture guide (10) of any preceding claim, wherein the flanking post (20) defines at least one channel (70) oriented in a first direction not perpendicular to the long axis (L) of the flanking post (20), and at least one additional channel (90) oriented in a second direction, different from the first direction, not perpendicular to the long axis (L) of the flanking post (20).

5. The suture guide (10) of any preceding claim, wherein at least one channel (50) of the flanking post (20) is perpendicular to the long axis (L) of the flanking post (20) so that it defines a horizontal suture needle guide path.

6. The suture guide (10) of any preceding claim, further comprising a second flanking post (30) disposed to the side of the guard (40) opposite from that of the first flanking post (20), each flanking post (20, 30) defining a plurality of channels (50, 70, 80, 90, 100) passing through its width, the channels (50, 70, 80, 90, 100) so oriented that:
a) the channels (50, 70, 80, 90, 100) define suture needle guide paths in one or more planes that do not intersect the tissue guard (40); and
b) at least one channel (70, 80, 90, 100) is not perpendicular to the long axis (L) of the flanking post (20, 30) so that it defines an oblique suture needle guide path.

7. The suture guide (10) of claim 6, wherein the channels (70, 80, 90, 100) of the two flanking posts (20, 30) are so aligned that one non-perpendicular channel (70, 80, 90, 100) from each of the first and second flanking posts (20, 30) are coaxial and together define an oblique suture needle guide path through the first post and the second post (20, 30).

8. The suture guide (10) of claim 6 or claim 7, wherein at least one channel (50) of each flanking post (20, 30) is perpendicular to the long axis (L) of the flanking post (20, 30) so that it defines a horizontal suture needle guide path.

## Patentansprüche

1. Fadenführung (10), die Folgendes umfasst:
einen Gewebeschutz (40), der dafür konfiguriert ist, über einem Gewebe angeordnet zu sein oder dieses zu stützen; und
eine flankierende Säule (20), die auf einer Seite des Gewebeschutzes (40) angeordnet ist,
wobei die flankierende Säule (20) eine Vielzahl von Kanälen (50, 70, 80, 90, 100) definiert,
die durch ihre Breite verlaufen, wobei die Kanäle (50, 70, 80, 90, 100) so ausgerichtet sind, dass:
a) die Kanäle (50, 70, 80, 90, 100) Nähnadelführungsbahnen in einer oder mehreren Ebenen definieren, die den Gewebeschutz (40) nicht schneiden; und
b) mindestens ein Kanal (70, 80, 90, 100) nicht senkrecht zu einer Längsachse (L) der flankierenden Säule (20) angeordnet ist, so dass er eine schräge Nähnadelführungsbahn definiert;
**dadurch gekennzeichnet, dass** die Säule (20) und der Schutz (40) durch ein Verbindungsstück (42) miteinander verbunden sind, das so einstellbar ist, dass es den Abstand zwischen der Säule (20) und dem Schutz (40) steuert.

2. Fadenführung (10) nach Anspruch 1, die ferner einen Schaft (44) umfasst, an dem der Schutz (40) und die Säule (20) so gekoppelt sind, dass die Säule (20) und der Schutz (40) in Bezug zueinander drehbar gemacht werden.

3. Fadenführung (10) nach einem der vorhergehenden Ansprüche, wobei der Schutz (40) eine konkave Form aufweist, die Konkavität den Nähnadelführungsbahnen zugewandt ist und Kanten der Schutzkonkavität sich nach hinten in Richtung der vordersten Nähnadelführungsebene, jedoch nicht in diese hinein, erstrecken.

4. Fadenführung (10) nach einem der vorhergehenden Ansprüche, wobei die flankierende Säule (20) mindestens einen Kanal (70), der in einer ersten Richtung nicht senkrecht zur Längsachse (L) der flankierenden Säule (20) ausgerichtet ist, und mindestens einen zusätzlichen Kanal (90) definiert, der in einer zweiten Richtung, die sich von der ersten Richtung unterscheidet, nicht senkrecht zur Längsachse (L) der flankierenden Säule (20) ausgerichtet ist.

5. Fadenführung (10) nach einem der vorhergehenden Ansprüche, wobei mindestens ein Kanal (50) der flankierenden Säule (20) senkrecht zu der Längsachse (L) der flankierenden Säule (20) angeordnet ist, so dass er eine horizontale Nähnadelführungsbahn definiert.

6. Fadenführung (10) nach einem der vorhergehenden Ansprüche, die ferner eine zweite flankierende Säule (30) umfasst, die auf der Seite des Schutzes (40) gegenüber der Seite der ersten flankierenden Säule (20) angeordnet ist, wobei jede flankierende Säule (20, 30) eine Vielzahl von Kanälen (50, 70, 80, 90, 100) definiert, die durch ihre Breite verlaufen, wobei die Kanäle (50, 70, 80, 90, 100) so ausgerichtet sind, dass:
a) die Kanäle (50, 70, 80, 90, 100) Nähnadelführungsbahnen in einer oder mehreren Ebenen definieren, die den Gewebeschutz (40) nicht schneiden; und
b) mindestens ein Kanal (70, 80, 90, 100) nicht senkrecht zu der Längsachse (L) der flankierenden Säule (20, 30) angeordnet ist, so dass er eine schräge Nähnadelführungsbahn definiert.

7. Fadenführung (10) nach Anspruch 6, wobei die Kanäle (70, 80, 90, 100) der beiden flankierenden Säulen (20, 30) so ausgerichtet sind, dass ein nicht-senkrechter Kanal (70, 80, 90, 100) von der ersten und zweiten flankierenden Säule (20, 30) koaxial angeordnet sind und zusammen eine schräge Nähnadelführungsbahn durch die erste Säule und die zweite Säule (20, 30) definieren.

8. Fadenführung (10) nach Anspruch 6 oder Anspruch 7, wobei mindestens ein Kanal (50) jeder flankierenden Säule (20, 30) senkrecht zu der Längsachse (L) der flankierenden Säule (20, 30) angeordnet ist, so dass er eine horizontale Nähnadelführungsbahn definiert.

## Revendications

1. Guide de suture (10), comportant :
un dispositif de protection de tissu (40) configuré pour recouvrir ou supporter le tissu ; et
une colonne latérale (20) disposée d'un côté du dispositif de protection de tissu (40), la colonne latérale (20) définissant une pluralité de canaux (50, 70, 80, 90, 100) traversant sa largeur, les canaux (50, 70, 80, 90, 100) étant orientés d'une telle manière que :
a) les canaux (50, 70, 80, 90, 100) définissent des trajets de guidage d'aiguille de suture dans un ou plusieurs plans qui ne croisent pas le dispositif de protection de tissu (40) ; et
b) au moins un canal (70, 80, 90, 100) n'est pas perpendiculaire par rapport à un axe long (L) de la colonne latérale (20), de telle sorte qu'il définit un trajet de guidage d'aiguille de suture oblique;
**caractérisé en ce que** la colonne (20) et le dispositif de protection (40) sont attachés l'un à l'autre par un connecteur (42) ajustable de telle manière à pouvoir contrôler l'espace entre la colonne (20) et le dispositif de protection (40).

2. Guide de suture (10) selon la revendication 1, comportant par ailleurs un arbre (44) auquel le dispositif de protection (40) et la colonne (20) sont accouplés de manière à rendre la colonne (20) et le dispositif de protection (40) rotatifs l'une par rapport à l'autre.

3. Guide de suture (10) selon l'une quelconque des revendications précédentes, dans lequel le dispositif de protection (40) a une forme concave, la concavité est orientée vers les trajets de guidage d'aiguille de suture, et les bords de la concavité du dispositif de protection s'étendent vers l'arrière par rapport au plan de guidage d'aiguille de suture le plus en avant, mais pas dans celui-ci.

4. Guide de suture (10) selon l'une quelconque des revendications précédentes, dans lequel la colonne latérale (20) définit au moins un canal (70) orienté dans une première direction qui n'est pas perpendiculaire par rapport à l'axe long (L) de la colonne latérale (20), et au moins un canal supplémentaire (90) orienté dans une deuxième direction, différente de la première direction, qui n'est pas perpendiculaire par rapport à l'axe long (L) de la colonne latérale (20).

5. Guide de suture (10) selon l'une quelconque des revendications précédentes, dans lequel au moins un canal (50) de la colonne latérale (20) est perpendiculaire par rapport à l'axe long (L) de la colonne latérale (20) de telle manière qu'il définit un trajet de guidage d'aiguille de suture horizontal.

6. Guide de suture (10) selon l'une quelconque des revendications précédentes, comportant par ailleurs une deuxième colonne latérale (30) disposée sur le côté du dispositif de protection (40) à l'opposé de celui de la première colonne latérale (20), chaque colonne latérale (20, 30) définissant une pluralité de canaux (50, 70, 80, 90, 100) traversant sa largeur, les canaux (50, 70, 80, 90, 100) étant orientés d'une telle manière que :
a) les canaux (50, 70, 80, 90, 100) définissent des trajets de guidage d'aiguille de suture dans un ou plusieurs plans qui ne croisent pas le dispositif de protection de tissu (40) ; et
b) au moins un canal (70, 80, 90, 100) n'est pas perpendiculaire par rapport à l'axe long (L) de la colonne latérale (20, 30), de telle sorte qu'il définit un trajet de guidage d'aiguille de suture oblique.

7. Guide de suture (10) selon la revendication 6, dans lequel les canaux (70, 80, 90, 100) des deux colonnes latérales (20, 30) sont alignés de telle manière qu'un canal non perpendiculaire (70, 80, 90, 100) de chacune des première et deuxième colonnes latérales (20, 30) sont coaxiaux et définissent ensemble un trajet de guidage d'aiguille de suture oblique au travers de la première colonne et de la deuxième colonne (20, 30).

8. Guide de suture (10) selon la revendication 6 ou la revendication 7, dans lequel au moins un canal (50) de chaque colonne latérale (20, 30) est perpendiculaire par rapport à l'axe long (L) de la colonne latérale (20, 30) de telle sorte qu'il définit un trajet de guidage d'aiguille de suture horizontal.
